Europäisches Patentamt

European Patent Office

Office européen des brevets

(19)

(11) Publication number: **0 180 171 B1**

(12) **EUROPEAN PATENT SPECIFICATION**

(45) Date of publication of patent specification: **08.04.92** (51) Int. Cl.5: **C12N 1/06**, G01N 33/53

(21) Application number: **85113657.2**

(22) Date of filing: **26.10.85**

(54) **Process for making a targeted cell susceptible to lysis by cytotoxic T lymphocytes.**

(30) Priority: **31.10.84 US 666880**

(43) Date of publication of application:
**07.05.86 Bulletin 86/19**

(45) Publication of the grant of the patent:
**08.04.92 Bulletin 92/15**

(84) Designated Contracting States:
**AT BE CH DE FR GB IT LI LU NL SE**

(56) References cited:
**EP-A- 0 103 139**

**CHEMICAL ABSTRACTS, vol. 102, no. 11, 18 March 1985, Columbus, OH (US); D.M.KRANZ et al., p. 440, no. 94055w**

**CHEMICAL ABSTRACTS, vol. 97, no. 9, 30 August 1982, Columbus, OH (US); D.DIALYNAS et al., p. 481, no. 70590u**

**CHEMICAL ABSTRACTS, vol. 81, no. 15, 14 October 1974, Columbus, OH (US); F.WISLOEFF et al., p. 317, no. 89587q**

(73) Proprietor: **MASSACHUSETTS INSTITUTE OF TECHNOLOGY**
**77 Massachusetts Avenue**
**Cambridge, MA 02139(US)**

(72) Inventor: **Kranz, David M.**
**45 Concord Ave., No. 12**
**Somerville, MA 02143(US)**
Inventor: **Tonegawa, Susumu**
**44 Broadlawn Park**
**Chestnut Hill, MA 02187(US)**
Inventor: **Eisen, Herman N.**
**9 Homestead St.**
**Waban, MA 02168(US)**

(74) Representative: **Bassett, Richard Simon et al**
**ERIC POTTER & CLARKSON St. Mary's Court**
**St. Mary's Gate**
**Nottingham NG1 1LE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

The U.S. government has rights in this invention by virtue of Grant Number P01 28900 and RO1 CA 15472 from the National Cancer Institute.

The body's natural defenses against many infections, particularly viral infections, and probably against many kinds of tumor cells are largely due to a set of thymus-derived lymphocytes called cytolytic or cytotoxic T lymphocytes, CTLs. CTLs exist in the body as clones. Each clone is specific for a particular antigenic structure on a target cell. Because of this specificity, the CTLs of a particular clone are able to lyse only cells with the corresponding antigenic structure on their surface, target cells. These antigenic structures, characteristically involving products of the genes of the major histocompatibility complex (MHC), may be categorized as either (1) foreign antigens that are recognized only in the context of class I self-MHC molecules or (2) allogenic MHC class I molecules.

The T lymphocyte receptor is a glycoprotein formed from two subunits which is located on and in the surface of a T lymphocyte. Each subunit is composed of a variable region which is specific for a particular antigenic structure, a joining region, a constant region which is common to all T lymphocyte receptors except for perhaps some differences between helper and cytotoxic T lymphocytes, a transmembrane region, and a cytoplasmic region. There are some differences in the amino acid sequences between species but substantial homology has been shown for at least the beta subunit of mouse and human origin. Other receptor-associated molecules, such as T3, are located in close proximity to the T lymphocyte receptor molecule and may act in conjunction with the T lymphocyte receptor in recognizing antigenic structures. The T lymphocyte receptor and associated molecules are reviewed by Stephen C. Meuer, Oreste Acuto, Thierry Hercend, Stuart F. Schlossman, and Ellis L. Reinherz in Ann. Rev. Immunol. 2:23-50 (1984).

In most individuals with viral infections or with cancer cells, the immune system is not capable of eradicating all the infected or abnormal cells. The reasons for the immune system's limited capacity are not presently known. With the discovery of the nucleotide and amino acid structure of the T lymphocyte receptor, new avenues for enhancing this capacity are likely to become available. One of these avenues is the present invention, which is the result of a totally unexpected discovery made during a series of experiments designed to elicit more knowledge about the recognition of the antigenic structures on cells for which the T lymphocyte is specific. The essence of the present invention is a process far making any selected cell a target for attack and lysis by the host's own cytotoxic T lymphocytes.

It is therefore an object of the present invention to provide a process for making any cell susceptible to lysis by cytotoxic T lymphocytes.

A further object of the present invention is to provide reagents for use in a process to make any cell susceptible to lysis by cytotoxic T lymphocytes.

A still further object of the present invention is to provide a process for in vivo or in vitro lysis of selected cells by cytotoxic T lymphocytes.

### SUMMARY OF THE INVENTION

The present invention is a process for making any selected or targeted cell susceptible to lysis by cytotoxic T lymphocytes (CTLs). The process consists of binding an antibody, directed against a region of the T lymphocyte receptor that is shared by most cytotoxic T lymphocytes or a receptor-associated molecule, to the surface of the cell to be lysed. The antibody, called anti-receptor antibody, may be bound directly by means of a chemical crosslinking agent such as glutaraldehyde, or indirectly, by means of a "linker" molecule bound to the CTL receptor. The "linker" may be another antibody against an antigen on the targeted cell surface, a hormone that binds to a receptor on the targeted cell surface, or a carbohydrate such as galactose which binds to receptors on liver cells. The process is applicable to both in vitro and in vivo situations. Possible uses for the process include selective lysis of tumor cells with specific tumor antigens on their surface or of viral infected cells with virus-specified antigens on their surface.

### BRIEF DESCRIPTION OF THE DRAWINGS

Fig. 1 is a schematic of the interaction between a CTL with specific surface receptors and a target cell with antibody directed against the CTL receptors bound to its surface.

Fig. 2 is a schematic of the interaction between a CTL with specific surface receptors, a target cell with specific surface molecules, and a bifunctional molecule consisting of an antibody specific for the receptor on the CTL surface and a molecule specific for the surface molecule on the target cell.

Fig. 3 is a schematic showing lysis of the target cell following the interaction described in either Fig. 1 or Fig. 2 with phagocytic scavenger cells (e.g. macrophage) ingesting the cellular debris.

### DETAILED DESCRIPTION OF THE PREFERRED EMBODIMENT

The present invention is a process for making a selected or targeted cell susceptible to lysis by a cytotoxic T lymphocyte (CTL).

In this process, the receptor on the CTL surface is recognized by and interacts with another molecule which is bound to the surface of the targeted cell. It is preferable to use the host's own CTLs, particularly if the interaction is to occur in vivo, but, in special circumstances, CTLs from other sources may also be used. Other sources would include tissue culture, another animal or the same species, or even an animal of a different species.

It is also preferrable to increase the number of "activated" or stimulated CTLs prior to interaction with the targeted cells. In vivo, the increase in number and the activation may be brought about by exposing the host to convenient, suitable antigens or combinations of antigens, e.g. allogenic cells such as cultured normal keratinocytes or buffy coat cells from human blood, attenuated virus vaccines (measles) and certain non-pathogenic virus. Combinations of more than one antigen are preferable for they are more likely than a single antigen to activate many CTL clones.

Lysis of the targeted cell requires binding of the target cell to the CTL, normally via the CTL's antigen specific receptor. The present invention is based on both the discovery of a practical and novel means for achieving this binding and on the unexpected discovery that the selected target cell can be lysed even when binding does not occur within the molecular context of a MHC gene product. Under normal conditions, the presence of the MHC gene product appears to be indispensible for recognition of a target cell by T lymphocytes. It is theorized that T lymphocytes with effector functions (helper and cytotoxic T lymphocytes) use the MHC gene products as their means for deciding whether or not a cell is foreign. Indeed, the conclusion drawn from one recent analysis by G. Berke, E. McVey, V. Hu, and W. R. Clark in the Journal of Immunology, 127:782-787 (1981) of lectin-dependent lysis of target cells by CTLs is that even in this putatively antigen-independent process the presence of class I MHC products is still necessary for target cell lysis.

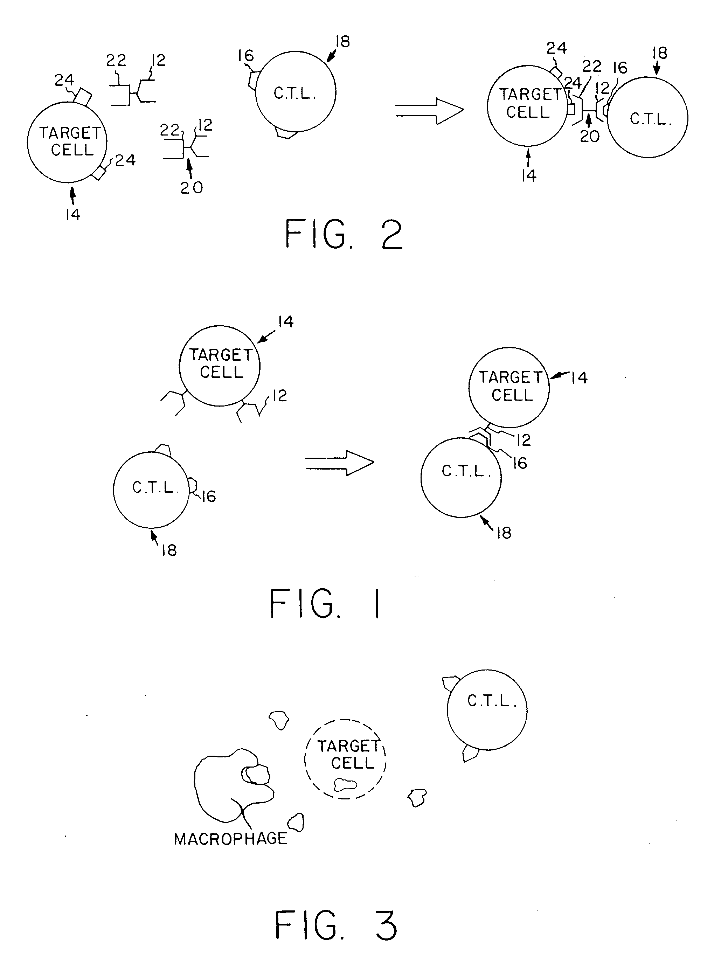

In the present invention, the CTL may be "bound" or brought into an interactive context with the target cell by means of a molecule that binds to the surfaces of both the CTL and the target cell. In one embodiment, shown in Fig. 1, an antibody 12 to the CTL receptor, an anti-receptor antibody, is bound to the target cell 14 by means of a crosslinking agent such as glutaraldehyde. The anti-receptor antibody 12 then binds to the CTL receptor 16 on the CTL 18, which lyses the target cell 14.

The preferred anti-receptor antibody is a monoclonal antibody directed against a region of the CTL's antigen-specific receptor which is shared by the corresponding receptors of most CTLs. Such a constant region is described by Saito et al. in Nature 309, 757-762 (1984) and Nature (November 1, 1984). A second anti-receptor antibody useful in the present invention is one directed against a cytotoxic T lymphocyte receptor-associated molecule, such as T3. The associated molecule must be located in close enough proximity to the CTL receptor to provide bridging between the CTL and the cell to be lysed so that the CTL receptor is located between the two linked cells.

Monoclonal anti-receptor antibodies of the desired specificity may be produced from hybridomas made from spleen cells isolated from immunized rats and fused with myeloma cells, wherein the rats are immunized using either

(1) CTL receptor protein isolated using antibody directed against the variable region of a particular CTL receptor,

(2) chemically synthesized peptides with a ten to twelve amino acid sequence corresponding to a part of the constant region of the CTL receptor, or

(3) polypeptides corresponding to most or all of a subunit of the receptor, made by recombinant DNA techniques.

In a second embodiment shown in Fig. 2, more suitable to an in vivo situation, lysis of a target cell 14 is produced by bridging the CTL to the target cell 14 by means of a "hybrid" molecule 20 made by joining an anti-receptor antibody 12 with another molecule 22 which selectively binds to a surface molecule 24 on the targeted cell 14. For example, a second antibody directed against a target cell surface antigen, such as a viral antigen or a tumor antigen, may be used as the molecule 22. A hormone, such as melanocyte stimulating hormone (MSH), which is selectively bound by a receptor on the targeted cell may also be used. The latter may be efficacious in the treatment of a melanoma type cancer, which is known to have specific MSH receptors on its surface. Other hormones, such as adrenal or thyroid hormones, might be useful in cases where removal of these glands is indicated. In most cases, the preferred molecule 22 to be attached to the anti-receptor antibody 12 is a protein. However, other molecules, such as carbohydrates, may also be used. An example of such a carbohydrate is galactose which binds to receptors on mammalian hepatocytes, described in "Carbohydrate Recognition Systems for Receptor-Mediated Pinocytosis" by E.F. Neufeld and G. Ashwell in The Biochemistry of Glycoproteins and Proteoglycans, ed. by William J. Lennarz (Plenum Press, N.Y., 1980) at pages 241-266.

The target cell specific protein may be linked to the anti-receptor antibody using any of the bifunctional crosslinking reagents known to those those skilled in the art. Examples of such cross-linking agents are glutaraldehyde and bifunctional sulfo-NHS-Esters such as 3,3'-Dithiobis(sulfosuccinimidyl propionate) described by J.V. Staros, in Biochemistry, 21: 3950-3955; Bis (sulfosuccinimidyl) suberate, described by D.P. Giedroc et al. in J. Biol. Chem., 258: 16-19 (1983); and N-succinimidyl-3-(2-pyridyldithio) proprionate described by A.R. Neurath, and N. Strick in J. Virol. Methods, 3: 155-165 (1981), which may be obtained from Pierce Chemical Co. The active or binding sites of the crosslinked molecules should be at the exposed ends so that they are able to interact with the molecules on the target cell and CTL surfaces.

For an in vivo situation wherein the crosslinked molecule is injected intravascularly, intraperitoneally, subcutaneously, or by other routes, the antibody molecules should either be selected from the classes or isotypes of antibodies which do not fix complement by the classical pathway, or be treated to remove those portions of the antibody constant region that are required to activate complement. The complement system consists of a group of protein molecules which can recognize certain sites on complexed or cell bound antibodies, attach to the complex or cell in a sequential fashion, and ultimately cause cellular lysis and inflammation. This is undesireable when it occurs in a non-specific or uncontrolled fashion. $IgG_4$, IgA and $Ig^E$ do not fix complement by the classical pathway. IgM, $IgG_1$, $IgG_2$ and $IgG_3$ do fix complement. Enzymatic treatment may be used to eliminate the complement-fixing portions of $IgG_1$, IgG2, $IgG_3$, and IgM while leaving the activated, antigen-binding sites intact.

An in vitro application of this method for promotion of CTL-mediated lysis would be in the selective treatment of cell populations removed from a patient or grown in tissue culture. An example would be to use a molecule consisting of an antibody against the CTL receptor and an antibody to an antigen found on the surface of tumor cells to eliminate tumor cells from bone marrow removed from a patient undergoing radiation treatment prior to re-introducing the bone marrow.

CTLs are found throughout the body: in tissues, the lymphatic system, and in the blood. Accordingly, it is possible to inject the appropriate crosslinked molecule into an animal or human and cause CTL mediated lysis of the targeted cells anywhere in the body. The protein molecules themselves have a relatively short half life. For example, human IgG has a half life of 23 days. The other human immunoglobulins have half lives of less than 6 days. See Davis, Dulbecco, Eisen, Ginsberg, Wood, and McCarty, Microbiology, p. 483 (2nd ed. 1973). If not bound and destroyed, the antibodies will circulate until they degrade and are removed, for example, by scavenger cells such as circulating macrophages, Kupffer cells in the liver, and alveolar macrophages in the lungs. The cellular debris resulting from CTL-mediated lysis may be disposed of in the same fashion, as shown in Fig. 3. In an in vivo situation, as with any drug, care must be exercised in the administration of the crosslinked molecule to avoid anaphylatic shock.

The present invention will be further understood from the following non-limiting example. All of the starting materials for this procedure are readily available to those skilled in the art from commercial or other sources.

Example

In the following detailed example, a monoclonal antibody (mAb) was produced against a CTL clone, clone 2C. This mAb was shown to block the CTL clone's cytolytic activity and to immunoprecipitate its CTL receptor, but not to block the activity or to precipitate the corresponding receptors of other CTL clones of similar specificity. The finding may be interpreted to mean the mAb was directed specifically against a portion of the variable region of the receptor unique to that CTL clone. The anti-CTL receptor mAb was purified and covalently attached to $^{51}$Cr-labeled cells. The labeled cells were then tested for susceptibility to lysis by CTL clones 2C and 2.1.1. Clone 2.1.1 is also specific for the product of a gene at the D end of the $H-2^d$ haplotype and, like clone 2C, it has its own unique cell surface molecule with the characteristics of the T lymphocyte receptor. The cells to which the mAb was attached included a human T cell lymphoma cell line, HPB-ALL , and murine cell lines that lack the $H-2^d$ haplotype of the natural targets for clone 2C; tumor cell lines EL4($H-2^b$), BW5147 ($H-2^k$), RI.I ($H-2^k$) and RI.E (a variant of RI.I which is negative for Class I surface antigens). A tumor cell line, P815 ($H-2^d$), which is normally lysed by clone 2C and clone 2.1.1 was used as a control.

Both clones 2C and 2.1.1 lysed the unmodified and modified (mAb coupled) P815 control cells to an equal extent. Cells lacking the $H-2^d$ haplotype (murine EL4 and human HPB-ALL cells) are lysed poorly, if at all by these clones. However, after attaching the mAb directed against clone 2C's receptor, murine EL 4 and human HPB-ALL were lysed by clone 2C but not by clone 2.1.1. Attaching mAbs directed against other CTL surface molecules such as LFA-1 and Thy-1 to EL-4 and BW5417 ($H-2^k$) cells did not render the cells susceptible to lysis by clone 2C.

Not only a human cell line but also cells without any detectable cell surface MHC class I molecules were shown to be convertible into target cells for a clone of murine CTLs using mAb to the CTL clone receptor. In this example, two cell lines which are not normally lysed by clone 2C were used. Rl.l is a cultured C58/J lymphoma (H-2$^k$) whose variant, Rl.E, does not produce beta$_2$-microglobulin and is devoid of cell surface class I molecules. After attachment of the mAb specific for the CTL clone 2C receptor, both Rl.l and Rl.E cells became susceptible to lysis by clone 2C. Neither modified nor unmodified Rl.l or Rl.E cells were lysed by the control clone 2.1.1.

The following materials and methods were used in the above described example.

Mice: BALB/c AnN (H-2$^d$) and BALB.B (H-2$^b$) mice were produced in the Center for Cancer Research, Massachusetts Institute of Technology.

Tumor Cell Lines: P815 (H-2$^d$), EL4(H-2$^b$), BW5147(H-2$^k$), Rl.l(H-2$^k$), Rl.E (a variant of Rl.l that is negative for class I surface antigens), HPB-ALL (a human T cell lymphoma), and X63.653 (a BALB/c derived myeloma) were all maintained in culture with RPMI 1640 containing 10% fetal calf serum, 10 mM HEPES, 2 mM L-glutamine, 100 Units penicillin/ml of culture fluid, 100 micrograms streptomycin/ml culture fluid, and $5 \times 10^{-5}$ M 2-mercaptoethanol.

Cloned CTL: Alloreactive CTL clones 2C, G4, and 2.1.1 were produced from spleen cells of BALB.B mice as described by M.V. Sitkovsky, M.S. Pasternack, and H.N. Eisen in J. Immunology, 124:1372-1376 (1982). The clones were maintained by weekly stimulation with irradiated (4000 rads) P815 cells plus supernatants from rat spleen cells maintained in culture for 48 hours in the presence of concanavalin A (Vector Laboratories, Burlingame, CA). All three clones lysed target cells that expressed a product of the D-end gene of the H-2$^d$ haplotype.

Monoclonal Antibodies: The mAb (1B2) that recognizes the T cell receptor of clone 2C was produced as described by D.M. Kranz, D.H. Sherman, M.V. Sitkovsky, M.S. Pasternack and H.N. Eisen in Proc. Nat. Acad. Sci. USA, 81:573-577, (1984). BALB/c AnN mice were immunized by intraperitoneal injection 6, times at 2-3 week intervals, each time with 10-20 x 10$^6$ cells from clone 2C. Four days after the final injection, spleen cells were harvested from the mice and fused with X63.653 myeloma cells using 50% polyethylene glycol. Cells were distributed into ten 24-well plates (Costar, Inc.) and grown in HAT (hypoxanthine, aminopterin, thymidine) selection media. After 2 to 3 weeks, culture supernatants were screened for their ability to block lysis of P815 target cells by clone 2C in a $^{51}$Cr-release assay, as described below.

Rat mAbs against lymphocyte function-associated antigen type 1 (4-16-1) and class I-H-2$^b$ surface antigens (3-18-8) were produced as previously described by Kranz et al, Proc. Nat. Acad. Sci. USA, 81:573-577 (1984). Rat mAb against Lyt-2 (3.155.2) described by M. Sarmiento, A. L. Glasebrook, and F. W. Fitch in J. Immunol., 125: 2665-1672 (1980) was a generous gift from Frank Fitch and Marian Sarmiento (University of Chicago).

Cytoxicity Assays: CTL-mediated target cell lysis was measured by a standard $^{51}$Cr-release assay as described by K. Grabstein in Selected Methods in Cellular Immunology, B.B. Mishell and S.M. Shiigi, eds. p. 124-137 (San Francisco: Freeman 1980). Various numbers of CTLs (in 100 microliters of media) were added to $2 \times 10^4$ $^{51}$Cr-labled target cells (in 100 microliters) and incubated at 37°C for 4 hours. Cells were pelleted by centrifugation, supernatants were assayed for radioactivity, and the percent specific $^{51}$Cr release was calculated from

$$100 \times \frac{(a-b)}{(t-b)}$$

where a is $^{51}$Cr release in the presence of CTL, b is the spontaneous release of $^{51}$Cr from labeled target cells in the absence of the CTL (less than 15%), and t is the total $^{51}$Cr content of the target cells. In some experiments, CTLs were preincubated with mAbs for 30 minutes prior to addition of $^{51}$Cr labeled target cells. All assays were performed in triplicate.

Purification and coupling of mAb 1B2: Antibodies were purified from ascites fluid that had been elicited by intraperitoneal injection of BALB/c mice with $5 \times 10^5$ viable 1B2 hybridoma cells. Lipoproteins were removed by adsorption to 0.25% sodium dextran sulfate and precipitation with 1.5% CaCl$_2$. The enriched gamma globulin fraction, obtained by precipitation with 50% saturated ammonium sulfate, was dialysed against 50 mM potassium phosphate, PH 8.0. Since mAb 1B2 contains gamma$_1$ heavy chains (determined by indirect radioimmune binding assays), the antibody was further purified by anion exchange chromatography on DEAE-cellulose. The pass-through from the DEAE-cellulose contained the antibody and was dialyzed against phosphate buffered saline, pH 7.4 (PBS).

Several methods of attaching mAb 1B2 to target cells were examined. In the mildest effective method, the mAb was crosslinked to the cell surface with 0.1% glutaraldehyde. In this procedure,

51Cr-labled cells were washed two times with RPMI-1640, resuspended in 0.25 ml of RPMI-1640 containing 0.2% gutaraldehyde, and 0.25 ml of mAb 1B2 (2 mg per ml in PBS) was added. The mixture was incubated on ice for 20 minutes and layered over a Ficol™ (Pharmacia Fine Chemicals) gradient. After centrifugation (3000 rpm, 15 min), the interphase (viable) cells were collected, washed three times with RPMI 1640 containing 10% fetal calf serum (FCS) and used in the cytoxicity assay.

Antibody was coupled to Affi-Gel™ 10 (Bio-Rad Laboratories) at about 5 mg per ml of packed gel for use in immunoprecipitation assays.

Labelling of Cell Surface Proteins with 125 I.: Cloned CTLs (2-3 x $10^7$) were washed three times with PBS and the following were added to the cell pellet: 100 microgram of lactoperoxidase, 1 mCi of Na $^{125}$I (New England Nuclear), and 50 microliters of 0.03% $H_2O_2$. Labeled cells were washed three times with PBS and extracted with 1 ml of 0.5% Nonidet P40 in 0.15 M NaCl, 0.02% $NaN_3$, 25 micromolar phenylmethylsulfonyl fluoride, 10 mM Tris-HCl, pH 7.2 (extraction buffer).

Immunoprecipitation and SDS/PAGE: $^{125}$I-labeled cell extracts (approximately $10^6$ cpm), containing 0.5% bovine serum albumin, were incubated with 50 microliters of 1B2-Affi-Gel 10™ for 3 hours on ice. The gel was then washed three times with the previously described extraction buffer and heated at 100°C in SDS/PAGE sample buffer, with or without 0.1 M 2-mercaptoethanol. Electrophoresis was performed in 10% polyacrylamide gels containing SDS according to the method of U.K. Laemmli, Nature (London) New Biol, 227:680-685 (1975). Gels were fixed, dried, and exposed to Kodak X-Omat XAR film™ using a Dupont Cronex Lighting Plus intensifying screen.

Although this invention has been described with reference to specific embodiments, it is understood that modifications and variations may occur to those skilled in the art. Such other embodiments and modifications are intended to fall within the scope of the appended claims.

## Claims

1. A process for making a targeted cell susceptible to lysis by a cytotoxic T lymphocyte comprising binding an antibody specific for cytotoxic T lymphocyte receptor determinants to said targeted cell.

2. The process of claim 1 wherein said receptor determinants are on the constant domain of a cytotoxic T lymphocyte receptor.

3. The process of claim 1 wherein said determinants are located on a cytotoxic T lymphocyte receptor-associated molecule.

4. The process of claim 3 wherein said receptor-associated molecule is T3.

5. The process according to claim 1 wherein said anti-receptor antibody is bound to said targeted cell by the non-antigen binding region of said antibody.

6. The process according to claim 5 wherein said anti-receptor antibody is bound to said targeted cell by means of a second molecule that specifically binds to the surface of said targeted cell.

7. The process according to claim 6 wherein said second molecule binding the antireceptor antibody to the targeted cell is a second antibody specific for an antigen on the surface of said targeted cell.

8. The process according to claim 6 wherein said second molecule is a hormone that specifically binds to a receptor on the surface of said targeted cell.

9. The process according to claim 6 wherein said second molecule is a carbohydrate that specifically binds to a receptor on the surface of said targeted cell.

10. The process according to claim 1 further comprising eliminating the complement fixing portion of said antibodies.

11. The process according to claim 7 further comprising eliminating the complement fixing portion of said antibodies.

12. The process according to claim 1 wherein said antibody is selected from the group of non-complement fixing antibodies consisting of IgG$_4$, IgA, and IgE.

13. The process according to claim 7 wherein said antibodies are selected from the group of non-complement fixing antibodies consisting of IgG$_4$, IgA, and IgE.

14. The process according to claim 6 wherein said anti-receptor antibody is bound to said second molecule using a bifunctional crosslinking reagent.

15. The process according to claim 6 wherein said second molecule specifically binds to a third molecule on the surface of said targeted cell selected from the group consisting of tumor antigens, viral antigens, major histocompatibility antigens, specific protein receptors, hormone receptors, and carbohydrate receptors.

16. A reagent for use in making a targeted cell susceptible to lysis by cytotoxic T lymphocytes comprising an antibody specific for T lymphocyte receptor determinants.

17. The reagent of claim 16 wherein said anti-receptor antibody is directed against a portion of a T lymphocyte receptor shared by cytotoxic T lymphocyte receptors.

18. The reagent of claim 16 wherein said anti-receptor antibody is directed against a portion of a cytotoxic T lymphocyte receptor-associated molecule.

19. The reagent of claim 16 further comprising a second molecule which specifically binds to the surface of said targeted cell.

20. The reagent of claim 19 wherein said second molecule is covalently hound to said anti-receptor antibody.

21. The reagent of claim 19 wherein said second molecule is selected from the group consisting of antibodies, hormones, and carbohydrates.

22. The reagent of claim 19 wherein said second molecule specifically binds to a third molecule on the surface of said targeted cell selected from the group consisting of tumor antigens, viral antigens, specific protein receptors, hormone receptors, and carbohydrate receptors.

23. A process for lysing targeted cells comprising binding an antibody directed against T lymphocyte receptor determinants shared by most cytotoxic T lymphocytes to said targeted cell and exposing said anti-receptor antibody-bound cells to cytotoxic T lymphocytes.

24. The process according to claim 23 wherein said anti-receptor antibody is bound to said targeted cell by means of a second molecule that specifically binds to the surface of said targeted cell.

25. The process according to claim 24 wherein said second molecule is selected from the group consisting of antibodies, hormones, and carbohydrates.

26. The process according to claim 23 wherein said anti-receptor antibody is directed against T3.

## Revendications

1. Procédé pour rendre une cellule ciblée sensible à la lyse par un T-lymphocyte cytotoxique, comprenant la liaison d'un anticorps spécifique pour ces déterminants de récepteurs du T-lymphocyte cytotoxique, à ladite cellule ciblée.

2. Procédé de la revendication 1, dans lequel lesdits déterminants de récepteurs sont sur le domaine constant d'un récepteur du T-lymphocyte cytotoxique.

3. Procédé de la revendication 1, dans lequel lesdits déterminants sont situés sur une molécule associée à un récepteur du T-lymphocyte cytotoxique.

4. Procédé de la revendication 3, dans lequel ladite molécule associée à un récepteur est T3.

5. Procédé selon la revendication 1, dans lequel ledit anticorps anti-récepteur est lié à ladite cellule ciblée par la région de liaison sans antigène dudit anticorps.

6. Procédé selon la revendication 5, dans lequel ledit anticorps anti-récepteur est lié à ladite cellule ciblée au moyen d'une deuxième molécule qui se lie de façon spécifique à la surface de ladite cellule ciblée.

7. Procédé selon la revendication 6, dans lequel ladite deuxième molécule liant l'anticorps anti-récepteur à la cellule ciblée est un deuxième anticorps spécifique pour un antigène sur la surface de ladite cellule ciblée.

8. Procédé selon la revendication 6, dans lequel ladite deuxième molécule est une hormone qui se lie de façon spécifique à un récepteur sur la surface de ladite cellule ciblée.

9. Procédé selon la revendication 6, dans lequel ladite deuxième molécule est un hydrate de carbone qui se lie de façon spécifique à un récepteur sur la surface de ladite cellule ciblée.

10. Procédé selon la revendication 1, comprenant de plus l'élimination de la portion de fixation du complément desdits anticorps.

11. Procédé selon la revendication 7, comprenant de plus l'élimination de la portion de fixation du complément desdits anticorps.

12. Procédé selon la revendication 1, dans lequel ledit anticorps est choisi dans le groupe d'anticorps de fixation sans complément consistant en IgG$_4$, IgA et IgE.

13. Procédé selon la revendication 7, dans lequel lesdits anticorps sont choisis dans le groupe d'anticorps de fixation sans complément consistant en IgG$_4$, IgA et IgE.

14. Procédé selon la revendication 6, dans lequel ledit anticorps anti-récepteur est lié à ladite deuxième molécule en utilisant un agent chimique de réticulation bifonctionnel.

15. Procédé selon la revendication 6, dans lequel ladite deuxième molécule se lie de façon spécifique à une troisième molécule sur la surface de ladite cellule ciblée choisie dans le groupe consistant en antigènes tumoraux, antigènes viraux, antigènes histocompatibles majeurs, récepteurs protéinés spécifiques, récepteurs hormonaux et récepteurs aux hydrates de carbone.

16. Agent chimique à utiliser pour rendre une cellule ciblée sensible à la lyse par les T-lymphocytes cytotoxiques, comprenant un anticorps spécifique pour des déterminants de récepteurs des T-lymphocytes.

17. Agent chimique de la revendication 16, dans lequel ledit anticorps anti-récepteur est destiné à une portion d'un récepteur d'un T-lymphocyte commune aux récepteurs des T-lymphocytes cytotoxiques.

18. Agent chimique de la revendication 16, dans lequel ledit anticorps anti-récepteur est destiné à une portion d'une molécule associée à un récepteur d'un T-lymphocyte cytotoxique.

19. Agent chimique de la revendication 16, comprenant de plus une deuxième molécule qui se lie de façon spécifique à la surface de ladite cellule ciblée.

20. Agent chimique de la revendication 19, dans lequel ladite deuxième molécule est liée par covalence audit anticorps anti-récepteur.

21. Agent chimique de la revendication 19, dans lequel ladite deuxième molécule est choisie dans le groupe consistant en anticorps, hormones et hydrates de carbone.

22. Agent chimique de la revendication 19, dans lequel ladite deuxième molécule se lie de façon spécifique à une troisième molécule sur la surface de ladite cellule ciblée choisie dans le groupe consistant en antigènes tumoraux, antigènes viraux, récepteurs protéinés spécifiques, récepteurs hormonaux et récepteurs aux hydrates de carbone.

23. Procédé de lyse de cellules ciblées comprenant la liaison d'un anticorps destiné aux déterminants du récepteur du T-lymphocyte qui sont communs à la plupart des T-lymphocytes cytotoxiques, à ladite cellule ciblée et l'exposition desdites cellules liées à l'anticorps anti-récepteur, aux T-lymphocytes cytotoxiques.

24. Procédé selon la revendication 23, dans lequel ledit anticorps anti-récepteur est lié à ladite cellule ciblée au moyen d'une seconde molécule qui se lie spécifiquement à la surface de ladite cellule ciblée.

25. Procédé selon la revendication 24, dans lequel ladite seconde molécule est choisie parmi le groupe constitué des anticorps, des hormones et des hydrates de carbone.

26. Procédé selon la revendication 23, dans lequel ledit anticorps anti-récepteur est destiné aux T3.

**Patentansprüche**

1. Verfahren zur Herstellung einer für eine Lyse durch einen zytotoxischen T-Lymphozyten anfälligen Target-Zelle, umfassend die Bindung eines für die Rezeptordeterminanten des zytotoxischen T-Lymphozyten spezifischen Antikörpers an die Target-Zelle.

2. Verfahren nach Anspruch 1, dadurch gekennzeichnet, daß sich die Rezeptordeterminanten auf der konstanten Domäne eines zytotoxischen T-Lymphozytenrezeptors befinden.

3. Verfahren nach Anspruch 1, dadurch gekennzeichnet, daß die Determinanten auf einem mit dem zytotoxischen T-Lymphozytenrezeptor assoziierten Molekül liegen.

4. Verfahren nach Anspruch 3, dadurch gekennzeichnet, daß es sich bei dem mit dem Rezeptor assoziierten Molekül um T3 handelt.

5. Verfahren nach Anspruch 1, dadurch gekennzeichnet, daß der Antirezeptorantikörper über den nicht-antigenbindenden Bereich des Antikörpers an die Target-Zelle gebunden ist.

6. Verfahren nach Anspruch 5, dadurch gekennzeichnet, daß der Antirezeptorantikörper mit Hilfe eines zweiten Moleküls mit spezifischer Bindung an die Oberfläche der Target-Zelle an die Target-Zelle gebunden ist.

7. Verfahren nach Anspruch 6, dadurch gekennzeichnet, daß es sich bei dem den Antirezeptorantikörper an die Target-Zelle bindenden zweiten Molekül um einen für ein Antigen auf der Oberfläche der Target-Zelle spezifischen zweiten Antikörper handelt.

8. Verfahren nach Anspruch 6, dadurch gekennzeichnet, daß es sich bei dem zweiten Molekül um ein Hormon mit spezifischer Bindung an einen Rezeptor auf der Oberfläche der Target-Zelle handelt.

9. Verfahren nach Anspruch 6, dadurch gekennzeichnet, daß es sich bei dem zweiten Molekül um ein Kohlenhydrat mit spezifischer Bindung an einen Rezeptor auf der Oberfläche der Target-Zelle handelt.

10. Verfahren nach Anspruch 1, bei dem zusätzlich der komplementfixierende Teil der Antikörper eliminiert ist.

11. Verfahren nach Anspruch 7, bei dem zusätzlich der komplementfixierende Teil der Antikörper eliminiert ist.

12. Verfahren nach Anspruch 1, dadurch gekennzeichnet, daß der Antikörper aus der Gruppe nicht-komplementfixierter Antikörper in Form von IgG$_4$, IgA und IgE ausgewählt ist.

13. Verfahren nach Anspruch 7, dadurch gekennzeichnet, daß die Antikörper aus der Gruppe nicht-komplementfixierender Antikörper in Form von IgG$_4$, IgA und IgE ausgewählt sind.

14. Verfahren nach Anspruch 6, dadurch gekennzeichnet, daß der Antirezeptorantikörper an das zweite Molekül unter Verwendung eines bifunktionellen Vernetzungsmittels gebunden ist.

15. Verfahren nach Anspruch 6, dadurch gekennzeichnet, daß das zweite Molekül speziell eine Bindung an ein drittes Molekül auf der Oberfläche der Target-Zelle, ausgewählt aus der Gruppe Tumorantigene, Virusantigene, Haupthistokompatibilitätsantigene, spezifische Proteinrezeptoren, Hormonrezeptoren und Kohlenhydratrezeptoren, eingeht.

16. Reagens zur Verwendung bei der Herstellung einer Target-Zelle, die gegen eine Lyse durch zytotoxische T-Lymphozyten empfindlich ist, umfassend einen für T-Lymphozytenrezeptordeterminanten spezifischen Antikörper.

17. Reagens nach Anspruch 16, dadurch gekennzeichnet, daß der Antirezeptorantikörper gegen einen Teil eines T-Lymphozytenrezeptors, an dem Rezeptoren eines zytotoxischen T-Lymphozyten teilhaben, gerichtet ist.

18. Reagens nach Anspruch 16, dadurch gekennzeichnet, daß der Antirezeptorantikörper gegen einen Teil eines mit einem Rezeptor eines zytotoxischen T-Lymphozyten assoziierten Moleküls gerichtet ist.

19. Reagens nach Anspruch 16, das zusätzlich ein zweites Molekül mit spezifischer Bindung an die Oberfläche der Target-Zelle umfaßt.

20. Reagens nach Anspruch 19, dadurch gekennzeichnet, daß das zweite Molekül kovalent an den Antirezeptorantikörper gebunden ist.

21. Reagens nach Anspruch 19, dadurch gekennzeichnet, daß das zweite Molekül aus der Gruppe Antikörper, Hormone und Kohlenhydrate ausgewählt ist.

22. Reagens nach Anspruch 19, dadurch gekennzeichnet, daß das zweite Molekül spezifisch eine Bindung an ein drittes Molekül auf der Oberfläche der Target-Zelle, ausgewählt aus der Gruppe Tumorantigene, Virusantigene, spezifische Proteinrezeptoren, Hormonrezeptoren und Kohlenhydratrezeptoren, gebunden ist.

23. Verfahren zum Lysieren von zu Target-Zellen gemachten Zellen durch Bindung eines gegen T-Lymphozytenrezeptordeterminanten, die den meisten zytotoxischen T-Lymphozyten gemeinsam sind gerichteten Antikörpers an die Target-Zelle und Einwirkenlassen zytotoxischer T-Lymphozyten auf die Antirezeptorantikörpergebundenen Zellen.

24. Verfahren nach Anspruch 23, dadurch gekennzeichnet, daß der Antirezeptorantikörper an die zur Target-Zelle gemachten Zelle mit Hilfe eines zweiten Moleküls, das eine spezifische Bindung an die Oberfläche der Target-Zelle eingeht, gebunden ist.

25. Verfahren nach Anspruch 24, dadurch gekennzeichnet, daß das zweite Molekül aus der Gruppe Antikörper, Hormone und Kohlenhydrate ausgewählt ist.

26. Verfahren nach Anspruch 23, dadurch gekennzeichnet, daß der Antirezeptorantikörper gegen T3 gerichtet ist.

FIG. 2

FIG. 1

FIG. 3